# EUROPÄISCHE PATENTSCHRIFT

(11) **EP 3 173 044 B1**
(45) Veröffentlichungstag und Bekanntmachung des Hinweises auf die Patenterteilung: **22.05.2019**
(21) Anmeldenummer: 16198105.5
(22) Anmeldetag: 10.11.2016
(51) Int. Cl.: A61B 18/14, A61B 18/00

(54) **CHIRURGISCHE VAPORISATIONSELEKTRODE**
SURGICAL VAPORISATION ELECTRODE
ÉLECTRODE CHIRURGICALE DE VAPORISATION

(30) Priorität: 26.11.2015 DE 102015015314
(43) Veröffentlichungstag der Anmeldung: 31.05.2017
(73) Patentinhaber: Olympus Winter & Ibe GmbH, 22045 Hamburg (DE)
(72) Erfinder: KNOPF, Christoph, 23554 Lübeck (DE)
(74) Vertreter: Ettmayr, Andreas

(56) Entgegenhaltungen:
- WO-A1-02/11635
- WO-A1-95/10978
- WO-A1-2005/122938
- DE-U1- 29 521 028
- US-A1- 2011 066 145
- US-A1- 2012 059 219
- US-A1- 2014 276 757
- US-A1- 2014 276 801

## Beschreibung

### Technisches Gebiet

Die Erfindung betrifft eine chirurgische Vaporisationselektrode.

### Stand der Technik

Aus dem Stand der Technik sind elektrische chirurgische Resektionswerkzeuge bekannt, bei deren Verwendung zum Resezieren Hochfrequenz(HF)-Wechselstrom durch den zu behandelnden Körperteil geleitet wird, um das entsprechende Gewebe gezielt lokal zu entfernen bzw. zu schneiden. Derartige Resektionswerkzeuge werden insbesondere eingesetzt, um z.B. adenomatöses Gewebe durch Vaporisation zu entfernen. Zu diesem Zweck wird an einer Elektrode eine HF-Spannung angelegt, die mittels geeigneter HF-Generatoren erzeugt und über entsprechende Zuführungen auf den Arbeitsteil der Elektrode aufgeschaltet wird, wobei derartige Elektroden je nach Ausbildung bipolar oder monopolar betrieben werden können.

Am häufigsten wird die monopolare Technik angewendet, wobei ein Pol der HF-Spannungsquelle über eine möglichst große Fläche als Neutralelektrode mit dem Patienten verbunden wird und das chirurgische Instrument (aktive Elektrode) den anderen Pol bildet. Der Strom fließt über den Weg des geringsten Widerstandes von der aktiven Elektrode zur Neutralelektrode, so dass in unmittelbarer Nähe der aktiven Elektrode die Stromdichte am höchsten ist. Folglich ist der thermische Effekt hier am ausgeprägtesten, aber auch anliegendes Gewebe wird durch Stromfluss erhitzt.

Bei der bipolaren Technik fließt der Strom im Gegensatz zur monopolaren Technik nur durch einen kleinen Teil des Körpers. Die lokalisierte Stromdichte bei der Bipolarelektrode bedingt eine rasche Erwärmung des die Elektrodenspitzen umgebenden Gewebes mit konsekutiver Vaporisation des Gewebewassers oder der das Gewebe umgebenden Spülflüssigkeit (Irrigierlösung, Saline).

Um die Elektrodenspitze bildet sich dabei eine dünne Gasschicht (Dampfpolster), welche bei ausreichend hoher Spannung (Plasmazündung) zu einem konstanten Plasma ionisiert werden kann. Die Energie des Plasmas überträgt sich auf die Zellen des zu resezierenden Gewebes und führt zu dessen lokal begrenzter Vaporisation. Durch Plasmavaporisation kann ein Gewebe schonender und effektiver getrennt bzw. entfernt werden als mit herkömmlicher Vaporisation (z.B. mittels monopolarer Vaporisation oder mittels Laserverdampfung), da die Plasmavaporisation nur in geringstem Maße den Kontakt zwischen Elektrode und Gewebe erfordert und ohne hohe Temperaturen auskommt ("kalte Vaporisation").

In der DE 102007054438 A1 ist eine chirurgische Vaporisationselektrode mit einem vorzugsweise halbkugelförmigen Elektrodenkopf beschrieben, wobei der Elektrodenkopf eine gekrümmte Arbeitsfläche aufweist und wenigstens eine Anschlussstelle, die an einen mit einem Isoliermantel umhüllten Zuleitungsdraht angeschlossen ist. Der die Anschlussstelle umgebende Oberflächenbereich des Elektrodenkopfes ist mit einer isolierenden Keramikabdeckung versehen. Die Abdeckung verhindert, dass sich an anderen Stellen des Elektrodenkopfes als der Arbeitsfläche Plasma bildet. Durch ihre keramische Ausführung ist die Abdeckung thermisch und chemisch hochstabil.

Aus der WO 2013/070311 A1 ist ein bipolares chirurgisches Resektionswerkzeug bekannt, welches entweder mit einer halbkugelförmigen oder mit einer ovalen Knopfelektrode ausgebildet ist. Letztere besitzt in Arbeitsrichtung eine axial Länge, die der der halbkugelförmigen Knopfelektrode entspricht, während ihre (laterale) Breitenausdehnung deutlich geringer ist, um so eine verbesserte Gewebe-Vaporisationsrate zu erzielen.

Bei den aus dem Stand der Technik bekannten Elektrodenköpfen ist die Arbeitsfläche des Elektrodenkopfes durch eine Kante begrenzt, bei einem halbkugeligen Elektrodenkopf beispielsweise entspricht diese Kante der Schnittlinie zwischen Kugeloberfläche und Kugelschnittfläche. Im Bereich dieser Kante herrscht lokale eine hohe Stromdichte, so dass hier verstärkt Vaporisation einsetzt. Während Gewebevaporisation je nach Einsatzfall meist vor allem im zentralen Bereich der Arbeitsfläche oder über einen größeren Flächenanteil der Arbeitsfläche erwünscht ist, treten Blasenbildung und Plasmaaktivität verstärkt im Kantenbereich und somit am Rand der Arbeitsfläche auf.

Je nach Größe und Form der Oberfläche der Vaporisationselektrode, der Einstellung des HF-Generators, der Gewebe-Impedanz, der Temperatur der Spülflüssigkeit sowie der Größe der Gewebekontaktfläche verändert sich zudem die Zündfähigkeit des Plasmas. Aufgrund der lokal höheren Stromdichte ist mit einer ersten Plasmazündung Am Elektrodenrand zu rechnen, was oft nicht erwünscht ist.

WO 95/10978 A1 offenbart eine chirurgische Vaporisationselektrode gemäß dem Oberbegriff des Anspruchs 1. WO 02/11635 A1 offenbart einen im wesentlichen kugeligen Elektrodenkopf mit im wesentlichen einheitlichem Oberflächenkrümmungsradius, US 2014/276757 A1 ein Elektrodeninstrument mit zwei kugeligen Elektrodenköpfen. US 2014/276801 A1 offenbart einen Elektrodenkopf mit abgerundetem Randbereich.

### Darstellung der Erfindung

Ausgehend von den vorgenannten Vorrichtungen des Standes der Technik liegt der vorliegenden Erfindung die Aufgabe zugrunde, eine Vorrichtung bereitzustellen, welche die genannten Nachteile nicht oder zumindest in geringerem Maße aufweist. Insbesondere soll eine Vaporisationselektrode geschaffen werden, welche die Vaporisation und Plasmazündung nicht besonders im Randbereich der Arbeitsfläche begünstigt.

Die Erfindung betrifft eine chirurgische Vaporisationselektrode gemäß Anspruch 1. Sie weist eine elektrische Anschlussleitung auf, welche einen mit der Anschlussleitung elektrisch verbundenen, elektrisch leitfähigen Elektrodenkopf aufweist. Der Elektrodenkopf kann aus den bereits für die Herstellung herkömmlicher Knopfelektroden bekannten Materialien gefertigt sein. Der Elektrodenkopf besitzt eine der Anschlussleitung zugewandten Rückfläche und einer der Anschlussleitung abgewandte, ebene oder konvex nach außen gewölbte Arbeitsfläche und einen gekrümmten Randbereich, in dem die Arbeitsfläche kantenfrei in die Rückfläche übergeht. Der lokale Krümmungsradius des Randbereichs ist an keinem Ort kleiner ist als ein Dreißigstel der Abmessung des Elektrodenkopfes in Richtung seiner maximalen Ausdehnung (also beispielsweise bei einem halbkugeligen Elektrodenkopf kleiner als ein Zehntel des Kugelradius).

Dass die Rückfläche der Anschlussleitung zugewandt ist, bedeutet im gegebenen Kontext, dass der Bereich der Verbindung zwischen der Anschlussleitung und dem Elektrodenkopf als Rückseite des Elektrodenkopfes definiert ist. Die Rückfläche ist also im Gegensatz zur Arbeitsfläche im wesentlichen in die Richtung des Elektrodenkopfes orientiert, in der die Anschlussleitung mit dem Elektrodenkopf zusammentrifft.

Dass die erfindungsgemäße chirurgische Vaporisationselektrode keine Kanten oder zu enge Krümmungsradien am Rand des Elektrodenkopfes aufweist, wird die Blasen- und Plasmabildung in diesem Bereich reduziert, und die Aktivität der Gas- bzw. Plasmabildung steht vor allem im chirurgisch genutzten Bereich der Arbeitsfläche zur Verfügung.

Vorzugsweise ist der lokale Krümmungsradius des Randbereichs an keinem Ort kleiner als ein Zwanzigstel, besonders bevorzugt an keinem Ort kleiner als ein Zehntel der Abmessung des Elektrodenkopfes in Richtung seiner maximalen Ausdehnung.

Vorzugsweise ist die Arbeitsfläche, beispielsweise im wesentlichen einem Kugelschnitt oder einem Elipsoid entsprechend, konvex gewölbt, die Arbeitsfläche kann aber auch eben ausgeführt werden. Durch die Wölbung kann der Winkel zwischen Elektrode bzw. Elektrodenmittelachse und Gewebe variieren, ohne die Resektionswirkung nennenswert zu beeinträchtigen. Dies ergibt eine einfachere Handhabung.

Gemäß einer bevorzugten Ausführungsform dient die Anschlussleitung als Haltearm zum Halten des Elektrodenkopfes und ist entsprechend steif ausgeführt.

Gemäß einer weiteren bevorzugten Ausführungsform weist die chirurgische Vaporisationselektrode eine die Rückfläche ganz oder teilweise bedeckende, isolierende Abdeckung auf. Da Plasma sich an allen elektrisch leitenden Oberflächen der Vaporisationselektrode und nicht nur an der Arbeitsfläche bilden kann, verhindert die isolierende Abdeckung vorteilhaft, dass sich Plasma auch an der Rückfläche nahe der Anschlussleitung und an deren Isolierummantelung bildet und somit einerseits die Anschlussleitung bzw. das sie umgebende Isoliermaterial beschädigt werden, und andererseits die Gewebevaporisation schlechter steuerbar wird. Besonders bevorzugt weist die isolierende Abdeckung ein keramisches Material auf. Durch die Hitzebeständigkeit des keramischen Materials kann die Anschlussleitung zum Elektrodenkopf optimal vor der Hitzeeinwirkung durch das Plasma geschützt werden.

Erfindungsgemäß weist die chirurgische Vaporisationselektrode einen gegenüber der Arbeitsfläche derart rückversetzten Elektrodenbereich auf, dass der rückversetzte Elektrodenbereich an zumindest zwei Seiten, vorzugsweise jedoch umlaufend, von einem Abschnitt der Arbeitsfläche berandet ist. Der rückversetzte Elektrodenbereich kann vorteilhaft als Aussparung in der Arbeitsfläche ausgeführt werden, indem die Aussparung beispielsweise gebohrt, gesägt, gefräst, gestanzt, geätzt oder als Spalt zwischen zwei oder mehreren nebeneinandergesetzten Elektrodenteilen erzeugt wird. Der rückversetzte Elektrodenbereich kann auch als (insbesondere konkave) Einwölbung der Oberfläche, die auch die Arbeitsfläche bildet, ausgeführt werden. Beispielsweise kann bei der Herstellung der Vaporisationselektrode der rückversetzte Elektrodenbereich durch ein entsprechendes Gussverfahren, mittels eines konvex geformten Stempels oder durch Ablation erzeugt werden.

Gemäß einer bevorzugten Ausführung der Erfindung weist mindesten eine Stelle des Überganges zwischen der Arbeitsfläche und dem rückversetzten Elektrodenbereich einen lokalen Krümmungsradius auf der kleiner ist, als der minimale lokale Krümmungsradius des Randbereichs. Besonders bevorzugt wird der Übergang zwischen Arbeitsfläche und dem rückversetzten Elektrodenbereich eine Kante auf. Aufgrund erhöhter lokaler Stromdichten erfolgt die Plasmazündung so bevorzugt am Übergang zwischen rückversetztem Elektrodenbereich und Arbeitsfläche.

Erfindungsgemäß ist ein in dem rückversetzten Elektrodenbereich angeordnetes, mit der Anschlussleitung elektrisch verbundenes, elektrisch leitfähiges Elektrodenlement vorgesehen, das ein freies Ende besitzt, wobei das elektrisch leitfähige Elektrodenlement an mindestens einem Ort, vorzugsweise an seinem freien Ende, einen lokalen Krümmungsradius aufweist, der kleiner ist, als der minimale lokale Krümmungsradius des Randbereichs zwischen Arbeitsfläche und Rückfläche. Aufgrund erhöhter lokaler Stromdichten erfolgt die Plasmazündung so bevorzugt im Bereich des elektrisch leitfähigen Elektrodenlements. Entsprechend ist das freie Ende des Elektrodenelements vorzugsweise konvex gewölbt oder konisch. Vorzugsweise ragt das freie Ende des elektrisch leitfähigen Elektrodenelements über den berandenden Abschnitt der Arbeitsfläche zumindest nicht hinaus bzw. ist gegenüber der Arbeitsfläche rückversetzt, um gegen mechanische Belastungen geschützt zu sein. Generell können das elektrisch leitfähige Elektrodenelement und die Arbeitsfläche aus einem gemeinsamen Werkstück herausgearbeitet werden, oder aber das elektrisch leitfähige Elektrodenelement wird separat gefertigt und in den rückversetzten Elektrodenbereich eingesetzt.

Eine derart, d.h. mit einem Ort bewusst starker lokaler Krümmung, vorteilhaft ausgestaltete Vaporisationselektrode ermöglicht die instantane Zündung des Plasmas am Übergang zwischen der Arbeitsfläche und dem rückversetzten Elektrodenbereich bzw. am beispielsweise dorn-, stift- oder zapfenartigen Elektrodenelement im rückversetzten Elektrodenbereich, da an kleinen Oberflächen bzw. an Oberflächen mit kleinen Krümmungsradien bei gleicher Spannung eine höhere elektrische Feldstärke generiert wird (Spitzenentladungseffekt). Die so erzeugte höhere Stromdichte am Elektrodenlement kann die für die Plasmazündung notwendige Gastasche um die Vaporisationselektrode schneller, stabiler und bei geringerem Energieaufwand auch unter ungünstigeren Bedingungen erzeugen, z.B. bei Aktivierung in freier Spülflüssigkeit.

Die Erfindung wird nachfolgend beispielhaft anhand der beigefügten schematischen Zeichnung näher erläutert. Die Zeichnungen sind nicht maßstabsgetreu; insbesondere entsprechen Verhältnisse der einzelnen Abmessungen zueinander aus Gründen der Anschaulichkeit nicht unbedingt den Abmessungsverhältnissen in tatsächlichen technischen Umsetzungen.

Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar.

### Kurze Beschreibung der Figuren

- Figur 1: zeigt eine Vergleichsbeispiel einer chirurgischen Vaporisationselektrode im Querschnitt.
- Figur 2: zeigt ein weiteres Vergleichsbeispiel einer chirurgischen Vaporisationselektrode im Querschnitt.
- Figur 3: zeigt ein Vergleichsbeispiel einer chirurgischen Vaporisationselektrode im Querschnitt, bei welcher ein rückversetzter Elektrodenbereich als Aussparung in der Arbeitsfläche ausgeführt ist.
- Figur 4a: zeigt ein Ausführungsbeispiel einer erfindungsgemäßen chirurgischen Vaporisationselektrode im Querschnitt, bei welcher in einem rückversetzten Elektrodenbereich ein elektrisch leitfähiges Elektrodenelement als Zündhilfe vorgesehen ist. Der Bereich des freien Endes des elektrisch leitfähigen Elektrodenelements ist zudem in einer zusätzlichen vergrößerten Teilansicht dargestellt.
- Figur 4b: zeigt die Vaporisationselektrode aus Fig. 4a in der Draufsicht, was gleichbedeutend ist mit der Projektion der Arbeitsfläche und des leitfähigen Elektrodenelements in der Projektionsebene A-A' in Fig. 4a.

### Bevorzugte Ausführung der Erfindung

Einander entsprechende Elemente sind in den Zeichnungsfiguren jeweils mit den gleichen Bezugszeichen bezeichnet.

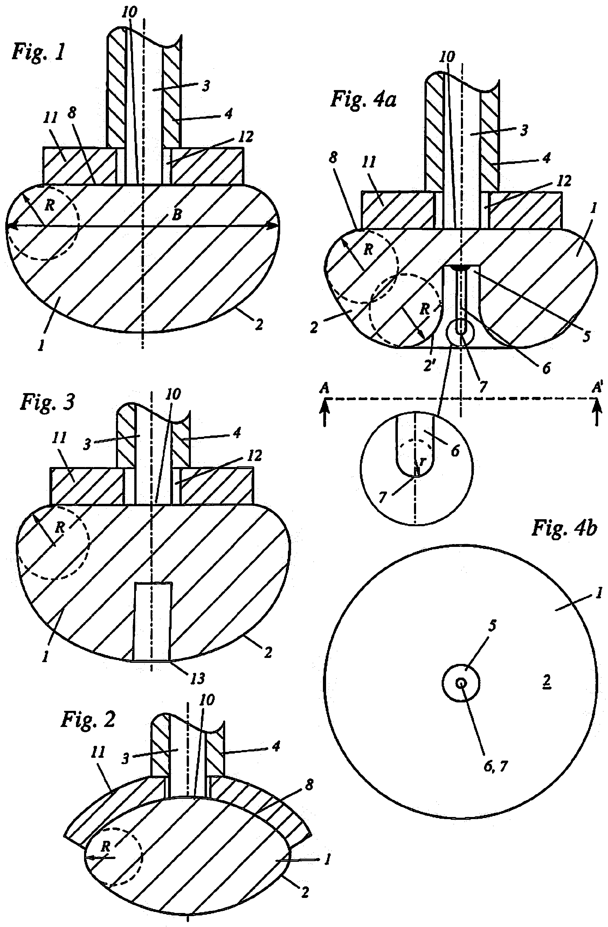

Fig. 1 zeigt den Elektrodenkopf 1 einer nicht erfindungsgemäßen chirurgischen Vaporisationselektrode, welche in ihren übrigen Bestandteilen aufgebaut sein kann wie herkömmliche aus dem Stand der Technik, beispielsweise DE 102007054438 A1, bekannte Vaporisationselektroden.

Die Arbeitsfläche 2 besteht aus einem geeigneten, hochtemperaturfesten Metall, wie es auch bei Vaporisationselektroden nach dem Stand der Technik verwendet wird. Im Ausführungsbeispiel ist die Arbeitsfläche 2 im wesentlichen halbkugelförmig ausgebildet, kann aber auch eine andere, beispielsweise ovaloide oder elipsoide, Grundform aufweisen.

Der Elektrodenkopf 1 wird über eine elektrische Anschlussleitung 3, die mit einer Isolierummantelung 4 aus Kunststoff versehen ist, mit Spannung versorgt. Die Anschlussleitung 3 und die gewölbte Arbeitsfläche 2 sind elektrisch miteinander verbunden.

Die elektrische Anschlussleitung 3 ist an einer Anschlussstelle 10 am Elektrodenkopf 1 angeschlossen, beispielsweise verschweißt oder verlötet. Die die Kugelschnittfläche bildende Rückfläche 8 des Elektrodenkopfes 1 ist eben ausgebildet und mit einer (grundsätzlich optionalen) isolierenden Abdeckung 11 aus keramischen Material abgedeckt. Die isolierende Abdeckung 11 weist eine Bohrung 12 auf, durch welche die elektrischen Anschlussleitung 3 hindurchtritt.

Der Übergangsbereich von der Arbeitsfläche 2 zur Rückfläche 8 besitzt keine Kante sondern ist abgerundet mit einem minimalen Krümmungsradius R, der im dargestellten Beispiel ca. ein Siebtel der dem Halbkugeldurchmesser (= doppeltem Halbkugelradius) entsprechenden Breite B, welche die Abmessung des Elektrodenkopfes 1 in Richtung seiner maximalen Ausdehnung ist, beträgt. Der verhältnismäßig große Krümmungsradius R verhindert, dass durch zu hohe lokale Stromdichten die Hauptaktivität der Elektrode an deren Rand vorliegt.

Die Abdeckung 11 kann auch näher an den gekrümmten Randbereich herangeführt sein oder diesen ganz oder teilweise bedecken.

Die Grundform des Elektrodenkopfes braucht nicht unbedingt halbkugelförmig zu sein. Bei der Ausführungsform in Fig. 2 weist der Elektrodenkopf 1 einen in der dargestellten Querschnittsebene (= Zeichenebene) linsenförmigen Querschnitt auf. Gemäß einer Variante kann der Elektrodenkopf 1 rotationssymmetrisch sein, so dass die Breite senkrecht zur Zeichenebene gleich der Breite in Zeichenebene ist. Gemäß einer weiteren Variante kann der Elektrodenkopf 1 in der Ebene senkrecht zur Zeichenebene aber auch langgestreckt sein, soweit eine Asymmetrie der Arbeitsfläche 2 in der chirurgischen Anwendung von Vorteil ist.

In die schalenartige Isolator-Abdeckung 11 ist der Elektrodenkopf 11 eingebettet.

Auch in Fig. 2 verhindert der verhältnismäßig große Krümmungsradius R am Übergang zwischen Arbeitsfläche 2 und Rückfläche 8, dass durch zu hohe lokale Stromdichten die Blasen- und Plasmabildung hauptsächlich am Elektrodenrand auftritt.

Bei dem in Fig. 3 dargestellten nicht erfindungsgemäßen Vergleichsbeispiel und den in Figuren 4a und 4b dargestellten dargestellten Ausführungsformen ist ein gegenüber der Arbeitsfläche 2 rückversetzter, durch eine Aussparung gebildeter Elektrodenbereich 5 vorgesehen, welcher umlaufend von einem Abschnitt der Arbeitsfläche 2 berandet ist. Bei der Aussparung kann es sich um eine einfache Bohrung handeln. Während in der Fig. 3 der umlaufende Rand des rückversetzten Elektrodenbereichs 5 eine Kante aufweist, iwölbt sich in Fig. 4a am Übergang zwischen dem rückversetzten Elektrodenbereich 5 und der Arbeitsfläche 2 die auch die Arbeitsfläche 2 bildende Oberfläche 2' nach innen. Der Krümmungsradius braucht hier nicht unbedingt, wie dargestellt, dem Krümmungsradius am Übergang zwischen Arbeitsfläche 2 und Rückfläche 8 zu entsprechen.

Abgesehen von dem rückversetzten Elektrodenbereich 5 entspricht das in Fig. 3 dargestellte Beispiel weitestgehend der Fig. 1. Die Kante 13 am Übergang zwischen rückversetztem Elektrodenbereich 5 und Arbeitsfläche 2 ermöglicht durch den sogenannten Spitzenentladungseffekt die instantane Zündung des Plasmas. Die lokal höhere Stromdichte kann die für die Plasmazündung notwendige Gastasche um die Vaporisationselektrode schneller, stabiler und bei geringerem Energieaufwand auch unter ungünstigeren Bedingungen erzeugen, z.B. bei Aktivierung in freier Spülflüssigkeit. Durch den geringeren erforderlichen Energieeintrag wird beim Zünden die Temperatur der Spülflüssigkeit weitaus geringfügiger erhöht, als bei einer herkömmlichen knopfförmigen Vaporisationselektrode.

In der Ausführungsform der Figuren 4a und 4b ist im rückversetzten Elektrodenbereich 5 ein ebenfalls mit der Anschlussleitung 3 elektrisch verbundenes, dornartig ausgeführtes, elektrisch leitfähiges Elektrodenelement 6 mit einem freien Ende 7 angeordnet. Das freie Ende 7 ist konvex gewölbt und weist einen lokalen Krümmungsradius r auf, der deutlich kleiner ist als der Krümmungsradius R am Übergang zwischen Arbeitsfläche 2 und Rückfläche 8.

Das elektrisch leitfähige Elektrodenelement 6 besitzt - bei Betrachtung in der Projektionsebene A-A', in der die Flächenausdehnung der Arbeitsfläche 2 gegenüber anderen Projektionsebenen maximal ist - eine Flächenausdehnung kleiner einem Zehntel der Flächenausdehnung der Arbeitsfläche 2. Die Projektionsebene A-A' ist eine beliebige zur Kugelschnittfläche 8 parallele Ebene. Das Elektrodenelement 6 ist an der Schweißstelle 9 in den rückversetzten Elektrodenbereich 5 mittels Laserschweißung engeschweißt, kann aber auch mittels anderer Fügetechniken, beispielsweise Löten, verbunden oder aber aus demselben Werkstück gebildet sein wie die Arbeitsfläche 2.

Um das Elektrodenelement 6 im rückversetzten Elektrodenbereich 5 bildet sich infolge der elektrischen Feldstärke bei geringerem Energieaufwand eine Gastasche für die Generation von konstantem Plasma. Das Elektrodenelement 6 ist so dimensioniert, dass sein freies Ende 7 gegenüber der Arbeitsfläche 2 zurückversetzt ist, also nicht über den berandenden Abschnitt der Arbeitsfläche 2 hinausragt. So ist es vor mechanischer Beschädigung geschützt.

## Patentansprüche

1. Chirurgische Vaporisationselektrode, aufweisend
eine elektrische Anschlussleitung (3),
einen mit der Anschlussleitung (3) elektrisch verbundenen, elektrisch leitfähigen Elektrodenkopf (1) mit einer der Anschlussleitung (3) zugewandten Rückfläche (8) und einer der Anschlussleitung (3) abgewandten, ebenen oder konvex nach außen gewölbten Arbeitsfläche (2),
einen gegenüber der Arbeitsfläche (2) derart rückversetzten Elektrodenbereich (5), dass der rückversetzte Elektrodenbereich (5) an zumindest zwei Seiten von einem Abschnitt der Arbeitsfläche (2) berandet ist, und
einen gekrümmten Randbereich, in dem die Arbeitsfläche (2) kantenfrei in die Rückfläche (8) übergeht,
wobei der lokale Krümmungsradius (R) des Randbereichs an keinem Ort kleiner ist als ein Dreißigstel der Abmessung des Elektrodenkopfes (1) in Richtung seiner maximalen Ausdehnung,
**dadurch gekennzeichnet,**
**dass** die Chirurgische Vaporisationselektrode ferner ein in dem rückversetzten Elektrodenbereich (5) angeordnetes, mit der Anschlussleitung elektrisch verbundenes, elektrisch leitfähiges Elektrodenlement (6) aufweist, das ein freies Ende (7) besitzt,
wobei das elektrisch leitfähiges Elektrodenlement (6) an mindestens einem Ort einen lokalen Krümmungsradius (r) aufweist, der kleiner ist, als der minimale lokale Krümmungsradius (R) des Randbereichs.

2. Chirurgische Vaporisationselektrode gemäß Anspruch 1,
wobei der lokale Krümmungsradius (R) des Randbereichs an keinem Ort kleiner ist als ein Zwanzigstel der Abmessung des Elektrodenkopfes (1) in Richtung seiner maximalen Ausdehnung.

3. Chirurgische Vaporisationselektrode gemäß Anspruch 2,
wobei der lokale Krümmungsradius (R) des Randbereichs an keinem Ort kleiner ist als ein Zehntel der Abmessung des Elektrodenkopfes (1) in Richtung seiner maximalen Ausdehnung.

4. Chirurgische Vaporisationselektrode gemäß einem der vorangehenden Ansprüche, wobei die Anschlussleitung (3) als Haltearm zum Halten des Elektrodenkopfes (1) dient.

5. Chirurgische Vaporisationselektrode gemäß einem der vorangehenden Ansprüche, ferner aufweisend eine die Rückfläche (8) ganz oder teilweise bedeckende, isolierende Abdeckung (11).

6. Chirurgische Vaporisationselektrode gemäß einem der vorangehenden Ansprüche, wobei mindesten eine Stelle des Überganges zwischen der Arbeitsfläche (2) und dem rückversetzten Elektrodenbereich (5) einen lokalen Krümmungsradius aufweist, der kleiner ist, als der minimale lokale Krümmungsradius (R) des Randbereichs.

## Claims

1. A surgical vaporization electrode, comprising
an electrical connecting line (3),
an electrically conductive electrode head (1) electrically connected to said connecting line (3) and having a rear surface (8) facing said connecting line (3) and also having a working surface (2) facing away from said connecting line (3) and being either planar or convexly outwardly curved,
an electrode region (5), which is set back with respect to said working surface (2), such that the setback electrode region (5) is bordered, on at least two sides thereof, by a section of said working surface(2)and
a curved boundary region, where said working surface (2) edgelessly merges into said rear surface (8), wherein the local radius of curvature (R) of said boundary region is not smaller at any location than one-thirtieth of the dimension of said electrode head (1) in a direction of its maximum extent,
**characterized in that**
the surgical vaporization electrode further comprises an electrically conductive electrode element (6), which is arranged in the setback electrode region (5) and is electrically connected to said connecting line and which has a free end (7),
wherein said electrically conductive electrode element (6) has, at at least one location thereof, a local radius of curvature (r) that is smaller than the minimum local radius of curvature (R) of the boundary region.

2. The surgical vaporization electrode according to claim 1,
wherein the local radius of curvature (R) of said boundary region is not smaller at any location than one-twentieth of the dimension of said electrode head (1) in the direction of its maximum extent.

3. The surgical vaporization electrode according to claim 2,
wherein the local radius of curvature (R) of said boundary region is not smaller at any location than one-tenth of the dimension of said electrode head (1) in the direction of its maximum extent.

4. The surgical vaporization electrode according to any of the preceding claims, wherein said connecting line (3) serves as a holding arm for holding said electrode head (1).

5. The surgical vaporization electrode according to any of the preceding claims, further comprising an insulating cover (11) completely or partially covering said rear surface (8).

6. The surgical vaporization electrode according to any of the preceding claims,
wherein at least one location at a transition between the working surface (2) and the setback electrode region (5) has a local radius of curvature, which is smaller than the minimum local radius of curvature (R) of said boundary region.

## Revendications

1. Électrode chirurgicale de vaporisation, présentant
un câble de raccordement électrique (3),
une tête d'électrode (1) électriquement conductrice, reliée électriquement au câble de raccordement (3), avec une surface arrière (8) tournée vers le câble de raccordement (3) et une surface de travail (2) opposée au câble de raccordement (3), plane ou courbée vers l'extérieur de manière convexe,
une zone d'électrode en retrait (5) par rapport à la surface de travail (2) de sorte que la zone d'électrode en retrait (5) est bordée au niveau d'au moins deux côtés par une section de la surface de travail (2), et
une zone de bord courbée, dans laquelle la surface de travail (2) passe sans arête à la surface arrière (8),
dans laquelle le rayon de courbure local (R) de la zone de bord n'est à aucun endroit inférieur à un trentième de la dimension de la tête d'électrode (1) en direction de son extension maximale,
**caractérisée en ce**
**que** l'électrode chirurgicale de vaporisation présente en outre un élément d'électrode électriquement conducteur (6), relié électriquement au câble de raccordement, agencé dans la zone d'électrode en retrait (5), qui dispose d'une extrémité libre (7),
dans laquelle l'élément d'électrode électriquement conducteur (6) présente à au moins un endroit un rayon de courbure local (r) qui est inférieur au rayon de courbure local minimum (R) de la zone de bord.

2. Électrode chirurgicale de vaporisation selon la revendication 1,
dans laquelle le rayon de courbure local (R) de la zone de bord n'est à aucun endroit inférieur à un vingtième de la dimension de la tête d'électrode (1) en direction de son extension maximale.

3. Électrode chirurgicale de vaporisation selon la revendication 2,
dans laquelle le rayon de courbure local (R) de la zone de bord n'est à aucun endroit inférieur à un dixième de la dimension de la tête d'électrode (1) en direction de son extension maximale.

4. Électrode chirurgicale de vaporisation selon l'une quelconque des revendications précédentes, dans laquelle le câble de raccordement (3) sert de bras de retenue pour retenir la tête d'électrode (1).

5. Électrode chirurgicale de vaporisation selon l'une quelconque des revendications précédentes, présentant en outre un recouvrement isolant (11), couvrant tout ou partie de la surface arrière (8).

6. Électrode chirurgicale de vaporisation selon l'une quelconque des revendications précédentes, dans laquelle au moins un endroit du passage entre la surface de travail (2) et la zone d'électrode en retrait (5), présente un rayon de courbure local, qui est inférieur au rayon de courbure local minimum (R) de la zone de bord.
